# FASCICULE DE BREVET EUROPEEN

(11) **EP 3 820 501 B1**
(45) Date de publication et mention de la délivrance du brevet: **14.09.2022**
(21) Numéro de dépôt: 19742016.9
(22) Date de dépôt: 15.07.2019
(51) Int. Cl.: A61K 38/28, A61P 7/12, A61K 9/08, A61K 47/10, A61K 47/26, A61K 9/00

(54) **FORMULATION THERMOSTABLE D'INSULINE HUMAINE A21G**
THERMOSTABILE FORMULIERUNG VON HUMANEM A21G-INSULIN
THERMOSTABLE FORMULATION OF A21G HUMAN INSULIN

(30) Priorité: 13.07.2018 FR 1856479
(43) Date de publication de la demande: 19.05.2021
(73) Titulaire: Adocia, 69003 Lyon (FR)
(72) Inventeur: CHAN, You-Ping, 69360 Ternay (FR); DI LORENZO, Fany, 69007 Lyon (FR)
(74) Mandataire: Tripoz, Inès
(86) Numéro de dépôt international: PCT/EP2019/069049
(87) Numéro de publication internationale: WO 2020/012040

(56) Documents cités:
- WO-A1-2007/104786
- WO-A1-2007/135118
- WO-A1-2009/021955

### SEQUENCE LISTING

<110> ADOCIA
<120> Formulation thermostable d'insuline A21G
<130> B1458PC00
<150> FR1856479
   <151> 2018-07-13
<160> 2
<170> BiSSAP 1.3.6
<210> 1
   <211> 21
   <212> PRT
   <213> Homo sapiens
<400> 1
   Gly Ile Val Glu Gln Cys Cys Thr Ser Ile Cys Ser Leu Tyr Gln Leu
   1               5                   10                  15
   Glu Asn Tyr Cys Gly
               20
<210> 2
   <211> 30
   <212> PRT
   <213> Homo sapiens
<400> 2
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Thr
               20                  25                  30

## Description

La présente invention telle que définie dans les revendications concerne une composition sous forme d'une solution aqueuse injectable, dont le pH est compris entre 7,2 et 8,0 comprenant au moins de l'insuline humaine A21G. Ladite composition présente une stabilité physique et/ou chimique améliorée(s), notamment à 40 et à 50°C. L'insuline humaine et ses analogues, produites par génie génétique depuis le début des années 80, ont révolutionné le traitement des patients diabétiques ayant besoin d'un traitement à base d'insuline.

L'observance (encore appelée« *compliance* ») et le traitement peuvent être encore améliorés par le choix des insulines, rapide ou basale, ainsi que par le choix des dispositifs d'injection, comme les stylos pré-remplis ou les pompes.

Dans la plupart des pays développés, l'accès à l'insuline est aisé et une personne diabétique peut, en général, mener une vie « normale ».

Toutes les insulines actuellement commercialisées doivent être conservées à une température comprise entre 2 et 8°C. Après la première utilisation, la plupart des insulines peuvent être conservées environ 1 mois à une température maximum de 30°C (Grajower et al. Diabetes Care 2003, 26,2665-2669).

Lors de l'utilisation d'un dispositif d'injection de type pompe, le réservoir comprenant l'insuline peut être en contact avec une surface dont la température est supérieure à 30°C. Ceci est notamment le cas lors de l'utilisation d'une pompe implantable dont le réservoir est placé sous la peau. Un des problèmes que vise à résoudre la présente invention peut être le développement d'une formulation d'insuline stable thermiquement, notamment à des températures supérieures à 30°C, et en particulier d'environ 37°C.

De plus, dans certaines régions du monde, la température moyenne est de l'ordre de 25°C et peut dépasser très régulièrement les 30°C et même localement atteindre 40°C à 45°C en journée. L'utilisation de pots en terre, courante dans certains pays chauds, permet de réduire la température moyenne de 3 à 8°C mais cela est souvent insuffisant pour une conservation de plus d'un mois de composition à base d'insuline (Ogle et al. Diabet. Med. 2016, 33, 1544-1553).

Ainsi, dans certains cas, l'insuline est difficilement accessible pour une partie de la population, du fait de son prix et des problèmes de maintien de la chaine du froid. Ceci est particulièrement criant dans le cas des régions à faible niveau de vie, ayant un climat chaud, voire tropical, et/ou éloigné de centres bénéficiant d'infrastructures permettant une bonne conservation de l'insuline.

Un des problèmes à résoudre est donc d'améliorer l'accès à l'insuline à un plus grand nombre de patients grâce à une formulation d'insuline ayant une stabilité à haute température améliorée, notamment par rapport aux insulines actuellement commercialisées. Il est également avantageux que cette formulation puisse être peu couteuse. Une formulation d'insuline stable thermiquement sur une durée relativement longue peut également être utile dans des régions dépourvues d'électricité, en zone de guerre ou en cas de catastrophe naturelle.

Il est connu que la stabilité chimique et physique des insulines peut être en partie dépendante de leur structure primaire ou séquence, du pH et de la nature des excipients.

A titres d'exemples choisis parmi des produits commerciaux, on peut citer les produits suivants.

L'insuline humaine, telle que commercialisée en flacon (encore appelé « vial ») et formulée à pH 7,4, ne permet pas d'obtenir une formulation stable à 40°C sur plus de 3 semaines car elle est instable physiquement. Ainsi, au-delà de cette durée, les spécifications de la pharmacopée européenne pour l'insuline ne sont plus respectées. En particulier, le taux d'agrégats chimiques dépasse rapidement les 2%, et le recouvrement est inférieur à 90%. A 50°C, l'insuline humaine n'est pas stable au-delà de quelques jours sur la base de la stabilité chimique. La stabilité physique à 50°C est inférieure à deux semaines. Le fabricant recommande de conserver ce produit au maximum 31 jours à une température ne dépassant pas 30°C.

L'insuline lispro, analogue de l'insuline humaine et formulée à pH 7,4, qui présente un résidu lysine en position B28 et un résidu proline en position B29, est une insuline prandiale commercialisée sous le nom de Humalog^{®}, et est stable physiquement moins d'une semaine à 50°C. Le fabricant recommande de conserver ce produit au maximum 28 jours à une température ne dépassant pas 30°C.

L'insuline glulisine, analogue de l'insuline humaine et formulée à pH 7,3, qui présente un résidu lysine en position B3 et un résidu acide glutamique en position B29, est une insuline prandiale commercialisée sous le nom de Apidra^{®}. Le fabricant recommande de conserver ce produit au maximum 28 jours à une température ne dépassant pas 25°C.

L'insuline glargine, analogue de l'insuline humaine et formulée à pH 4,0, qui présente un résidu glycine en position A21 et deux résidus arginines en position B30 et B31, est une insuline basale commercialisée sous le nom de Lantus^{®}, et est stable physiquement moins d'une semaine à 50°C. Le fabricant recommande de conserver ce produit au maximum 28 jours à une température ne dépassant pas 30°C.

De nombreuses études portent sur le rôle des excipients dans l'amélioration de la stabilité des compositions comprenant de l'insuline. Elles décrivent plus spécifiquement que le zinc et les conservateurs peuvent jouer un rôle important à cet égard. Pour une revue sur la stabilité de l'insuline, il est possible de se référer aux publications de Brange J. et al. suivantes :
- Insulin Structure and Stability. In: Wang Y.J., Pearlman R. (eds) Stability and Characterization of Protein and Peptide Drugs. Pharmaceutical Biotechnology, vol 5. Springer, Boston, MA) 1993,
- Chemical Stabiliy of Insulin. 1. Pharmaceutical Research 1992, 9, 715-726,
- Chemical Stability of Insulin. 2. Pharmaceutical Research 1992, 9, 727-734,
- Chemical Stability of Insulin. 3. Acta. Pharm. Nord. 1992, 4, 149-158,
- Chemical Stability of Insulin. 4. Acta. Pharm. Nord. 1992, 4, 209-222.

Des voies connues de dégradation chimique de l'insuline humaine sont notamment la déamidation des asparagines en B3 et A21, la formation d'agrégats covalents, et la rupture et réarrangement des ponts di-sulfures. Ces modes de dégradations sont dépendants, au moins en partie, du pH de la formulation et de la présence d'excipients. La déamidation en A21 est prépondérante à pH acide alors que celle en B3 est plus importante à pH neutre.

S'agissant de la stabilité physique, il est connu que les insulines peuvent former des fibrilles ou agrégats, en particulier en chauffant ou en secouant. Ces fibrilles ou agrégats pouvant être immunogéniques, ils doivent donc être évités.

L'obtention d'une insuline physiquement et chimiquement stable sur une plus longue durée à des température supérieures 30°C, notamment à 40 et/ou à 50°C, est un défi car la dégradation chimique et la formation de fibrilles ou agrégats proviennent de phénomènes distincts dont les mécanismes, cinétiques et facteurs déclenchants ne sont pas tous connus. En effet, même si les principales impuretés issues de la dégradation des insulines sont connues, il reste de nombreuses impuretés non-identifiées et par conséquent, issues de mécanisme(s) inconnu(s).

La compréhension de quelques uns des modes de dégradation permet d'apporter certaines solutions pour améliorer la stabilité physique et/ou chimique. Cependant, des améliorations significatives ayant un intérêt pratique, notamment conduisant à une stabilité physique supérieure à un mois à 40°C et permettant au moins l'insulinothérapie post-prandiale n'ont pas été décrites à ce jour.

Dans ce contexte, une formulation d'insuline prandiale présentant une bonne stabilité physique et/ou chimique à des températures supérieures à 30°C, comme 40 et 50°C serait très intéressante. En particulier une formulation d'insuline prandiale « thermostable » présentant les caractéristiques suivantes serait un produit très avantageux par rapport à tous les produits actuellement commercialisés :
- stabilité physique et chimique d'au moins un mois à 40°C et d'au moins 2 semaines à 50°C,
- bonne compatibilité avec les conservateurs anti-microbiens,
- utilisation possible à la fois en vials (flacons) et/ou en cartouches,
- profil pharmacocinétique similaire à celui de l'insuline humaine à la même concentration, et
- facilité de fabrication.

De façon surprenante, la demanderesse a mis au point une composition, sous forme de solution aqueuse injectable, comprenant une insuline humaine A21G à un pH compris entre 7,2 et 8,0 présentant une stabilité physique et/ou chimique à 40°C durant au moins 3 semaines et/ou à 50°C pendant au moins une semaine.

On notera que la précision de la mesure de la valeur de pH est de± 0,1.

Selon un mode de réalisation, le pH des compositions est compris entre 7,2 et 8,0.

Selon un autre mode de réalisation, le pH des compositions est compris entre 7,2 et 7,8.

Selon encore un autre mode de réalisation, le pH des compositions est compris entre 7,2 et 7,6.

Selon un mode de réalisation, la composition est stable physiquement et/ou chimiquement au moins un mois (c'est à dire 4 semaines) à 40°C et au moins deux semaines à 50°C.

Selon un mode de réalisation, la composition est stable physiquement au moins un mois à 40°C et au moins deux semaines à 50°C.

Selon un mode de réalisation, la composition est stable chimiquement au moins un mois (c'est à dire 4 semaines) à 40°C et au moins deux semaines à 50°C.

Selon un mode de réalisation, la composition est stable chimiquement au moins 4 semaines à 40°C.

Selon un mode de réalisation, la composition est stable chimiquement au moins 8 semaines à 40°C.

Selon un mode de réalisation, la composition est stable chimiquement au moins 12 semaines à 40°C.

Selon un mode de réalisation, la composition est stable chimiquement au moins deux semaines à 50°C.

Selon un mode de réalisation, la composition est stable physiquement au moins 8 semaines à 40°C.

Selon un mode de réalisation, la composition est stable physiquement au moins 12 semaines à 40°C.

Selon un mode de réalisation, la composition est stable physiquement au moins 16 semaines à 40°C.

Selon un mode de réalisation, la composition est stable physiquement au moins 4 semaines à 50°C.

Selon un mode de réalisation, la composition est stable physiquement au moins 6 semaines à 50°C.

Selon un mode de réalisation, la composition est stable physiquement au moins 8 semaines à 50°C.

Selon un mode de réalisation, la composition est stable physiquement au moins 12 semaines à 50°C.

On entend par « formulation thermostable », une formulation présentant une stabilité physique et chimique d'au moins 4 semaines à 40°C et d'au moins 2 semaines à 50°C.

On entend par « composition stable chimiquement », une composition dont le recouvrement en insuline est d'au moins 90 % et dont le taux de HMWP (« agrégats ») est inférieur à 2%.

On entend par « composition stable physiquement », des compositions qui satisfont aux critères de l'inspection visuelle décrite dans la pharmacopée européenne, américaine et internationale, c'est-à-dire des compositions qui sont claires et qui ne contiennent pas de particules visibles, mais également incolores.

On entend par « solution aqueuse injectable » des solutions à base d'eau qui répondent aux conditions des pharmacopées EP et US.

Les compositions sous forme d'une solution aqueuse injectable selon l'invention sont des solutions limpides. On entend par « solution limpide », des compositions qui satisfont aux critères décrits dans les pharmacopées américaines et européenne concernant les solutions injectables. Dans la pharmacopée US, les solutions sont définies dans la partie <1151> faisant référence à l'injection (<1>) (faisant référence à <788> selon USP 35 et précisé dans <788> selon USP 35 et dans <787>, <788> et <790> USP 38 (à partir du 1^{er} aout 2014), selon USP 38). Dans la pharmacopée européenne, les solutions injectables doivent remplir les critères donnés dans les sections 2.9.19 et 2.9.20.

On entend par « mensuelle » ou « un mois », une durée de 4 semaines.

On entend par « bimensuelle » une durée de 2 semaines.

Dans les exemples, on entend par « impureté dB3 » une insuline dans laquelle l'asparagine située en troisième position de la chaine B est déamidée et convertie en acide aspartique.

Dans les exemples, on entend par « impureté disoB3 » une insuline dans laquelle l'asparagine située en troisième position de la chaine B est déamidée et convertie en un acide isoaspartique.

L'insuline humaine A21G est une insuline qui présente la même séquence que l'insuline humaine mais dans laquelle l'asparagine en position A21 est mutée en glycine. Autrement dit cette insuline humaine A21G présente une séquence identique à celle de l'insuline humaine, à l'exception de la position A21 où un résidu glycine remplace le résidu asparagine présent dans l'insuline humaine.

La séquence de la chaine A est SEQ ID NO :1 GIVEQCCTSICSLYQLENYCG et la séquence de la chaine B est SEQ ID NO :2 FVNQHLCGSHLVEALYLVCGERGFFYTPKT.

Cette insuline humaine A21G est le métabolite principal de l'insuline glargine, commercialisée sous le nom de Lantus^{®} (SANOFI) (Bolli et al. Diabetes Care 2012, 35, 2626-2630). Cette insuline a une durée d'action similaire à celle de l'insuline humaine et peut ainsi être utilisée au moment des repas, il s'agit donc d'une insuline dite « prandiale ».

L'obtention d'une composition sous forme de solution aqueuse injectable présentant des propriétés de stabilités physique et chimique améliorées par rapport à celles des insulines prandiales décrites dans l'art antérieur à des températures supérieures à 30°C et à un pH proche de 7, est remarquable car il est bien connu de l'homme de l'art que les propriétés de stabilité physique et chimique sont très difficiles à prédire, tout particulièrement dans le cas de combinaisons.

L'invention concerne encore des contenants comprenant la composition selon l'invention. Ledits contenant pouvant être des flacons, des cartouches, des seringues préremplies, et des réservoirs. Lesdits contenants pouvant être inclus ou compris dans des stylos à injection ou dans des pompes à insuline.

L'invention concerne également des compositions pour leur utilisation en tant que médicament.

L'invention concerne encore des compositions pour leur utilisation dans le traitement du diabète.

Selon un mode de réalisation il s'agit du diabète de type I.

Selon un mode de réalisation il s'agit du diabète de type II.

L'invention concerne également des formulations pharmaceutiques comprenant de telles compositions.

L'invention concerne également l'utilisation d'une composition selon l'invention.

La divulgation concerne encore une méthode de traitement du diabète comprenant l'administration d'une composition selon l'invention. Elle concerne plus particulièrement une méthode comprenant l'administration d'une composition selon l'invention en tant qu'insuline prandiale.

Selon un aspect il s'agit d'une méthode de traitement destiné à traiter les êtres humains.

L'invention concerne également, une composition selon l'invention destinée à être utilisée dans une méthode de traitement du diabète caractérisée en ce qu'elle est administrée en bolus avant les repas.

Elle concerne plus particulièrement une composition destinée à être utilisée dans une méthode de traitement du diabète caractérisée en ce qu'elle est administrée en tant qu'insuline prandiale.

Selon un mode de réalisation l'insuline humaine A21G est sous forme hexamèrique.

Dans la présente demande 100 U d'insuline humaine A21G correspondent à 3,5 mg.

Dans un mode de réalisation, la concentration d'insuline humaine A21G est comprise entre 240 et 6000 µM ou entre 40 et 1000 U/mL.

Dans un mode de réalisation, la concentration d'insuline humaine A21G est comprise entre 240 et 3000 µM ou entre 40 et 500 U/mL.

Dans un mode de réalisation, la concentration d'insuline humaine A21G est 600 µM ou 100 U/mL.

Dans un mode de réalisation, la concentration d'insuline humaine A21G est 1200 µM ou 200 U/mL.

Dans un mode de réalisation, la concentration d'insuline humaine A21G est 1800 µM ou 300 U/mL.

Dans un mode de réalisation, la concentration d'insuline humaine A21G est 2400 µM ou 400 U/mL.

Dans un mode de réalisation, la concentration d'insuline humaine A21G est 3000 µM ou 500 U/mL.

Dans un mode de réalisation, la concentration d'insuline humaine A21G est 3600 µM ou 600 U/mL.

Dans un mode de réalisation, la concentration d'insuline humaine A21G est 4200 µM ou 700 U/mL.

Dans un mode de réalisation, la concentration d'insuline humaine A21G est 4800 µM ou 800 U/mL.

Dans un mode de réalisation, la concentration d'insuline humaine A21G est 5400 µM ou 900 U/mL.

Dans un mode de réalisation, la concentration d'insuline humaine A21G est 6000 µM ou 1000 U/mL.

Dans un mode de réalisation, les compositions selon l'invention comprennent en outre des sels de zinc.

Dans un mode de réalisation, le zinc provient de ZnCl₂ ou de ZnO.

Dans un mode de réalisation, les compositions selon l'invention comprennent des sels de zinc à une concentration comprise entre 50 et 600 µM pour 100 U d'insuline humaine A21G.

Dans un mode de réalisation, les compositions selon l'invention comprennent des sels de zinc à une concentration comprise entre 100 et 500 µM pour 100 U d'insuline humaine A21G.

Dans un mode de réalisation, les compositions selon l'invention comprennent des sels de zinc à une concentration comprise entre 150 et 400 µM pour 100 U d'insuline humaine A21G.

Dans un mode de réalisation, les compositions selon l'invention comprennent des sels de zinc à une concentration comprise entre 200 et 350 µM pour 100 U d'insuline humaine A21G.

Dans un mode de réalisation, les compositions selon l'invention comprennent des sels de zinc à une concentration comprise entre 200 et 300 µM pour 100 U d'insuline humaine A21G.

Dans un mode de réalisation, les compositions selon l'invention comprennent des sels de zinc à une concentration de 230 µM pour 100 U d'insuline humaine A21G.

Dans un mode de réalisation, les compositions selon l'invention comprennent en outre au moins un tampon.

Dans un mode de réalisation, les compositions selon l'invention comprennent un tampon choisi dans le groupe constitué par un tampon acétate de sodium et un tampon Tris, abrégé de trishydroxyméthylaminométhane.

Dans un mode de réalisation, les compositions selon l'invention comprennent un tampon Tris.

Dans un mode de réalisation, le trishydroxyméthylaminométhane est présent à une concentration comprise entre 2 et 100 mM.

Dans un mode de réalisation, le trishydroxyméthylaminométhane est présent à une concentration comprise entre 4 et 80 mM.

Dans un mode de réalisation, le trishydroxyméthylaminométhane est présent à une concentration comprise entre 5 et 50 mM.

Dans un mode de réalisation, le trishydroxyméthylaminométhane est présent à une concentration comprise entre 6 et 40 mM.

Dans un mode de réalisation, le trishydroxyméthylaminométhane est présent à une concentration comprise entre 6 et 20 mM.

Dans un mode de réalisation, les compositions selon l'invention comprennent de l'arginine.

Dans un mode de réalisation, l'arginine est présente à une concentration comprise entre 5 et 100 mM.

Dans un mode de réalisation, l'arginine est présente à une concentration comprise entre de 10 et 90 mM.

Dans un mode de réalisation, l'arginine est présente à une concentration comprise entre 20 et 80 mM.

Dans un mode de réalisation, l'arginine est présente à une concentration comprise entre 30 et 70 mM.

Dans un mode de réalisation, l'arginine est présente à une concentration comprise entre 40 et 60 mM.

Dans un mode de réalisation, les compositions selon l'invention comprennent de l'arginine et un tampon tris.

Dans un mode de réalisation, les compositions selon l'invention comprennent en outre des conservateurs.

Dans un mode de réalisation, les conservateurs sont des conservateurs phénoliques.

Dans un mode de réalisation, les conservateurs phénoliques sont choisis dans le groupe constitué par le m-crésol et le phénol, seuls ou en mélange.

Dans un mode de réalisation, la concentration en conservateur(s) phénolique(s) est comprise entre 10 et 100 mM.

Dans un mode de réalisation, la concentration en conservateur(s) phénolique(s) est comprise entre 15 et 100 mM.

Dans un mode de réalisation, la concentration en conservateur(s) phénolique(s) est comprise entre 20 et 75 mM.

Dans un mode de réalisation, la concentration en conservateur(s) phénolique(s) est comprise entre 20 et 60 mM.

Dans un mode de réalisation, le conservateur est le phénol.

Dans un mode de réalisation, la concentration en phénol est comprise entre 30 et 75 mM.

Dans un mode de réalisation, la concentration en phénol est comprise entre 40 et 60 mM.

Dans un mode de réalisation, la concentration en phénol est de 50 mM.

Dans un mode de réalisation, le conservateur est le m-crésol.

Dans un mode de réalisation, la concentration en m-crésol est comprise entre 15 et 50 mM.

Dans un mode de réalisation, la concentration en m-crésol est comprise entre 20 et 30 mM.

Dans un mode de réalisation, la concentration en m-crésol est de 25 mM.

Dans un mode de réalisation, la composition comprend un mélange de m-crésol et de phénol.

Dans un mode de réalisation, la concentration totale en m-crésol et en phénol est comprise entre 20 et 75 mM.

Selon un mode de réalisation la composition est dépourvue d'agent chélateur de zinc.

Par « agent chélateur du zinc », on entend un agent qui soit capable de complexer le zinc. Cet agent peut avoir un constante de stabilité de liaison au métal (« metal binding stability constant ») logK pour le zinc d'au moins 4,5 déterminée à 25°C.

Les constantes de stabilité de liaison au métal listées dans le « National Institute of Standards and technology reference database 46 (Critically Selected Stability Constants of metal Complexes) peuvent être utilisées. Cette base de données liste les constantes logK déterminées à 25°C. Par conséquent, la conformité d'un agent chélatant au sens de la présencte invention peut être déterminé sur la base de sa constate de stabilité de liaison pour le zinc, mésuréé à 25°C, et tel que décrit dans cette base de données.

Parmi les agents chélateurs de zinc on peut citer les anions organiques polydentates.

Selon un mode de réalisation, l'agent chélateur est choisi parmi les composés suivants : l'EDTA (logK = 14,5), le citrate, (logK = 4,93), l'EGTA (logK = 12,6), le pyrophosphate (logK = 8,71) et l'alginate (logK = 6,91).

Selon un autre mode de réalisation, l'agent chélateur est un composé comprenant une paire d'électrons libres ou une densité électronique peremttant une interaction avec le zinc ionique. De tels composés peuvent être des amines polydentates. Celles-ci peuvent être choisies parmi les composés suivants : l'éthylène diamine, les substances aromatiques ou hétéroaromatiques, en particulier celles comprenant une unité imidazole, comme l'histidine (logK ) 6,91).

Dans un mode de réalisation, les compositions selon l'invention comprennent en outre un tensioactif.

Dans un mode de réalisation, le tensioactif est choisi dans le groupe constitué par le Poloxamer 188, et les polysorbates, en particulier le Tween^{®} 20, encore appelée Polysorbate 20, et le Tween^{®} 80, encore appelé Polysorbate 80.

Dans un mode de réalisation, le tensioactif est choisi parmi les polysorbates.

Dans un mode de réalisation la concentration en polysorbate est comprise de 4 et 20 µM (4 µM ≤ concentration en polysorbate ≤ 20 µM).

Dans un mode de réalisation, le tensioactif est choisi parmi le Tween^{®} 20, encore appelée Polysorbate 20, et le Tween^{®} 80, encore appelé Polysorbate 80.

Dans un mode de réalisation, le tensioactif est le Tween^{®} 20, encore appelée Polysorbate 20.

Dans un mode de réalisation la concentration de Tween^{®} 20 est comprise entre 4 et 20 µM.

Dans un mode de réalisation la concentration de Tween^{®} 20 est comprise entre 5 et 10 µM.

Dans un mode de réalisation la concentration de Tween^{®} 20 est de 8 µM.

Dans un mode de réalisation la concentration Poloxamer 188 est comprise entre 0,5 et 2,5 mg/ml.

Dans un mode de réalisation la concentration Poloxamer 188 est comprise entre 0,8 et 1,6 mg/ml.

Dans un mode de réalisation la concentration Poloxamer 188 est de 1,2 mg/ml.

Selon un mode de réalisation la composition est dépourvue de polysorbate 80.

Selon un mode de réalisation la composition est dépourvue d'au moins un agent chélateur de zinc et de polysorbate 80.

Dans un mode de réalisation, la composition comprend du Tween^{®} 20 et du tampon Tris.

Dans un mode de réalisation, l'invention concerne une composition sous forme d'une solution aqueuse injectable, dont le pH est compris de 7,2 à 7,6, comprenant au moins l'insuline humaine A21G un tensioactif et un tampon. Selon un mode de réalisation il s'agit de Tween^{®} 20 et de tampon Tris.

Dans un mode de réalisation, l'invention concerne une composition sous forme d'une solution aqueuse injectable, dont le pH est 7,4, comprenant au moins l'insuline humaine A21G un tensioactif et un tampon. Selon un mode de réalisation il s'agit de Tween^{®} 20 et de tampon Tris.

Les compositions selon l'invention peuvent en outre comprendre des additifs tels que des agents de tonicité, encore appelés agents osmotiques.

Dans un mode de réalisation, les agents de tonicité sont choisis dans le groupe constitué par la glycérine, le chlorure de sodium, le mannitol, le trehalose et la glycine.

Dans un mode de réalisation, l'agent de tonicité est la glycérine.

Dans un mode de réalisation, la composition comprend une concentration en agent osmotique comprise entre 20 et 500 mM.

Dans un mode de réalisation, la concentration en agent de tonicité est comprise entre 30 et 400 mM.

Dans un mode de réalisation, la concentration en agent de tonicité est comprise entre 50 et 250 mM.

Dans un mode de réalisation, la composition selon l'invention contient de 3,5 mg/mL à 10,5 mg/mL d'insuline humaine A21G, 50 mM de phénol, 50 à 250 mM de glycérine à un pH de 7,4. Cette composition peut contenir en outre de 200 à 900 µM de zinc.

Dans un mode de réalisation, la composition selon l'invention contient 3,5 mg/mL d'insuline humaine A21G, 50 mM de phénol, 150 à 250 mM de glycérine, 10 mM de tampon TRIS à un pH de 7,4. Cette composition peut contenir en outre 200 à 250 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 3,5 mg/mL d'insuline humaine A21G, 50 mM de phénol, 50 à 100 mM de glycérine, 50 mM de tampon TRIS à un pH de 7,4. Cette composition peut contenir en outre 200 à 250 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 7,0 mg/mL d'insuline humaine A21G, 50 mM de phénol, 150 à 250 mM de glycérine, à un pH de 7,4. Cette composition peut contenir en outre 400 à 500 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 10,5 mg/mL d'insuline humaine A21G, 50 mM de phénol, 150 à 250 mM de glycérine, à un pH de 7,4. Cette composition peut contenir en outre 600 à 750 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient de 3,5 mg/mL à 10,5 mg/mL d'insuline humaine A21G, 25 mM de m-crésol, 150 à 250 mM de glycérine à un pH de 7,4. Cette composition peut contenir en outre de 200 à 1000 µM de zinc.

Dans un mode de réalisation, la composition selon l'invention contient 3,5 mg/mL d'insuline humaine A21G, 50 mM de phénol, 150 à 250 mM de glycérine, 10 mM de tampon TRIS à un pH de 7,4. Cette composition peut contenir en outre 200 à 250 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 3,5 mg/mL d'insuline humaine A21G, 25 mM de m-crésol, 150 à 250 mM de glycérine, 10 mM de tampon TRIS à un pH de 7,4. Cette composition peut contenir en outre 200 à 250 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 7,0 mg/mL d'insuline humaine A21G, 25 mM de m-crésol, 150 à 250 mM de glycérine, à un pH de 7,4. Cette composition peut contenir en outre 400 à 500 µM de zinc. Cette composition peut en outre comprendre du polysorbate 20, en particulier à 8 µM ou du poloxamer 188, en particulier à une concentration de 1,2 mg/ml.

Dans un mode de réalisation, la composition selon l'invention contient 10,5 mg/mL d'insuline humaine A21G, 25 mM de m-crésol, 150 à 250 mM de glycérine, à un pH de 7,4. Cette composition peut contenir en outre 600 à 750 µM de zinc. Cette composition peut en outre comprendre du polysorbate 20, en particulier à 8 µM ou du poloxamer 188, en particulier à une concentration de 1,2 mg/ml.

Selon un mode de réalisation, les compositions décrites ci-dessus comprennent du polysorbate 20, en particulier à 8 µM.

Dans un mode de réalisation, l'invention concerne une composition sous forme d'une solution aqueuse injectable comprenant au moins l'insuline humaine A21G et un agoniste au récepteur du GLP-1 ou un analogue du GLP-1.

Dans un mode de réalisation, les GLP-1, les analogues de GLP-1, ou les GLP-1 RA sont choisis dans le groupe constitué par l'exenatide (Byetta^{®}, ASTRA-ZENECA), le lixisenatide (Lyxumia^{®}, SANOFI), leurs analogues ou dérivés et leurs sels pharmaceutiquement acceptables.

L'exenatide et de lixisénatide respectivement décrits dans les demandes US2004/0023871 et WO0104156 sont généralement considérés comme des agonistes au récepteur du GLP-1.

Dans un mode de réalisation, le GLP-1, analogue de GLP-1, ou GLP-1 RA est l'exenatide ou Byetta^{®}, ses analogues ou dérivés et leurs sels pharmaceutiquement acceptables.

Dans un mode de réalisation, GLP-1, analogue de GLP-1, ou GLP-1 RA est le lixisenatide ou Lyxumia^{®}, ses analogues ou dérivés et leurs sels pharmaceutiquement acceptables.

Dans un mode de réalisation, l'invention concerne une composition sous forme d'une solution aqueuse injectable, dont le pH est compris entre 7,2 et 8,0, comprenant au moins l'insuline humaine A21G et un agoniste au récepteur du GLP-1 ou un analogue du GLP-1.

Selon un mode de réalisation ledit agoniste au récepteur du GLP-1 est l'exénatide.

Selon un autre mode de réalisation ledit agoniste au récepteur du GLP-1 est le lixisénatide.

Dans un mode de réalisation, l'invention concerne une composition sous forme d'une solution aqueuse injectable, dont le pH est compris de 7,2 à 7,6, comprenant au moins l'insuline humaine A21G et un agoniste au récepteur du GLP-1 ou un analogue du GLP-1.

Selon un mode de réalisation ledit agoniste au récepteur du GLP-1 est l'exénatide.

Selon un autre mode de réalisation ledit agoniste au récepteur du GLP-1 est le lixisénatide.

Dans un mode de réalisation, l'invention concerne une composition sous forme d'une solution aqueuse injectable, dont le pH est 7,4, comprenant au moins l'insuline humaine A21G et un agoniste au récepteur du GLP-1 ou un analogue du GLP-1.

Selon un mode de réalisation ledit agoniste au récepteur du GLP-1 est l'exénatide.

Selon un autre mode de réalisation ledit agoniste au récepteur du GLP-1 est le lixisénatide.

Dans un mode de réalisation, la composition comprend en outre un composé nicotinique ou un de ses dérivés.

Dans un mode de réalisation, la composition comprend du nicotinamide.

Dans un mode de réalisation, la concentration de nicotinamide est comprise entre 10 et 160 mM.

Dans un mode de réalisation, la concentration de nicotinamide est comprise entre20 et 150 mM.

Dans un mode de réalisation, la concentration de nicotinamide est comprise entre40 et 120 mM.

Dans un mode de réalisation, la concentration de nicotinamide est comprise entre60 et 100 mM.

Dans un mode de réalisation, la composition pharmaceutique comprend en outre au moins un promoteur d'absorption choisi parmi les promoteurs d'absorption, les promoteurs de diffusion ou les agents vasodilatateurs, seuls ou en mélange.

Les promoteurs d'absorption incluent, sans se limiter, les surfactants, par exemple, les sels biliaires, les sels d'acides gras ou les phospholipides ; les agents nicotiniques, comme les nicotinamides, les acides nicotiniques, la niacine, la niacinamide, la vitamine B3 et leurs sels ; les inhibiteurs de la trypsine pancréatique ; les sels de magnésium ; les acides gras polyinsaturés ; le phosphatidylcholine didécanoyl ; les aminopolycarboxylates ; la tolmétine ; le caprate de sodium ; l'acide salicylique ; l'acide oléique ; l'acide linoléique ; l'acide eicosapentaénoïque (EPA) ; l'acide docosahexaénoïque (DHA) ; l'acide benzylique ; les donneurs de monoxyde d'azote, par exemple, la 3-(2-hydroxy-1-(1-méthyléthyl)-2-nitrosohydrazino)-1-propanamine, la N-éthyl-2-(1-éthyl-hydroxy-2-1-nitrosohydrazino)-éthanamine, ou la S-nitroso-N-acétylpenicillamine ; les acides biliaires, la glycine sous sa forme conjuguée à un acide biliaire ; l'ascorbate de sodium, l'ascorbate de potassium ; le salicylate de sodium, le salicylate de potassium, l'acide acétyl-salicylique, l'acide salicylosalicylique, l'acétylsalicylate d'aluminum, le salicylate de choline, le salicylamide, l'acétylsalicylate de lysine ; l'exalamide ; le diflunisal ; l'éthenzamide ; l'EDTA ; seul ou en mélange.

Dans un mode de réalisation, la composition pharmaceutique comprend en outre au moins un promoteur de diffusion. Des exemples de promoteur de diffusion incluent, sans se limiter, les glycosaminoglycanases, par exemple la hyaluronidase.

Dans un mode de réalisation, la composition pharmaceutique comprend en outre au moins un agent vasodilatateur.

Dans un mode de réalisation, la composition pharmaceutique comprend en outre au moins un agent vasodilatateur provoquant une hyperpolarisation en bloquant les canaux ioniques de calcium.

Dans un mode de réalisation, l'agent vasodilatateur provoquant une hyperpolarisation en bloquant les canaux ioniques de calcium est l'adénosine, un agent hyperpolarisant dérivé de l'endothélium, un inhibiteur de la phosphodiestérase de type 5 (PDE5), un agent d'ouverture des canaux potassiques ou toute combinaison de ces agents.

Dans un mode de réalisation, la composition pharmaceutique comprend en outre au moins un agent vasodilatateur à médiation par AMPc.

Dans un mode de réalisation, la composition pharmaceutique comprend en outre au moins un agent vasodilatateur à médiation par GMPc.

Dans un mode de réalisation, la composition pharmaceutique comprend en outre au moins un agent vasodilatateur choisi dans le groupe comprenant les agents vasodilatateurs qui agissent en provoquant une hyperpolarisation en bloquant les canaux ioniques de calcium, les agents vasodilatateurs à médiation par AMPc, et les agents vasodilatateurs à médiation par GMPc.

Le au moins un agent vasodilatateur est chosi dans le groupe comprenant, les donneurs de monoxyde d'azote, par exemple, la nitroglycérine, le dinitrate d'isosorbide, le mononitrate d'isosorbide, le nitrate d'amyl, l'érythrityle, le tétranitrate, et le nitroprussiate) ; la prostacycline et ses analogues, par exemple l'époprosténol sodique, l'iloprost, l'époprostenol, le tréprostinil ou le selexipag ; l'histamine, la 2-méthylhistamine, la 4-méthylhistamine; la 2-(2-pyridyl)éthylamine, la 2-(2-thiazolyl)éthylamine ; la papavérine, le chlorhydrate de papavérine ; le minoxidil ; la dipyridamole ; l'hydralazine ; l'adénosine, l'adénosine triphosphate; l'uridine trisphosphate ; le GPLC ; la L-carnitine ; l'arginine ; la prostaglandine D2 ; les sels de potassium ; et dans certains cas, les antagonistes des récepteurs α1 et α2 ,par exemple, le prazosine, la phénoxybenzamine, la phentolamine, la dibénamine, le chlorhydrate de moxisylyte et la tolazoline), le bétazole, le dimaprit ; les agonistes des récepteurs β2, par exemple, l'isoprotérénol, la dobutamine, l'albutérol, la terbutaline, l'aminophylline, la théophylline, la caféine ; l'alprostadil, l'ambrisentan ; la cabergoline ; la diazoxide ; le mesilate de dihydralazine ; le chlorhydrate de diltiazem ; l'énoximone ; le chlorhydrate de flunarizine ; l'extrait de Ginkgo biloba ; le lévosimendan ; la molsidomine ; l'oxalate acide de naftidrofuryl ;le nicorandil ;la pentoxifylline ; le chlorure de phenoxybenzamine ; le piribédil base ; le mesilate de piribédil ; le regadenoson monohydrate ; le riociguat ; le sildenafil citrate, le tadalafil, le chlorhydrate trihydraté de vardenafil ; le chlorhydrate de trimetazidine ; la trinitrine ; le chlorhydrate de vérapamil ; les antagonistes des récepteur à l'endothéline, par exemple l'avanafil et le bosentran monohydrate ; et les inhibiteurs des canaux calciques,par exemple, l'amlodipine, l'aranidipine, l'azelnidipine, la barnidipine, la benidipine, la cilnidipine, la clévidipine, l'isradipine, l'efonidipine, la felodipine, la lacidipine, la lercanidipine, la manidipine, la nicardipine, la nifedipine, la nilvadipine, la nimodipine, la nisoldipine, la nitrendipine, la prandipine ; seul ou en mélange.

Les compositions selon l'invention peuvent comprendre en outre tous les excipients conformes aux Pharmacopées, notamment EP et/ou US, et compatibles avec les insulines utilisées aux concentrations d'usage.

Selon un mode de réalisation, la composition peut être sous forme solide ou lyophilisée. Cette composition peut alors servir à reconstituer une solution ou une formulation.

Les modes d'administration envisagés sont par voie intraveineuse, sous-cutanée, intradermique ou intramusculaire.

Selon un mode de réalisation particulier le mode d'administration est la voie sous-cutanée.

Les voies d'administration transdermique, orale, nasale, vaginale, oculaire, buccale, pulmonaire sont également envisagées.

L'invention concerne également une pompe, implantable ou transportable, comprenant une composition selon l'invention.

L'invention concerne encore l'utilisation d'une composition selon l'invention destinée à être placée dans une pompe, implantable ou transportable.

Dans un mode de réalisation, la pompe est une pompe implantable sous la peau. Parmi ces pompes on peut citer les pompes permettant une administration d'insuline par voie intra-péritonéale.

Dans un mode de réalisation, la pompe délivrant la composition selon l'invention par voie intra-péritonéale est la pompe MiniMed Medtronic MIP2007.

L'invention concerne également des formulations unidoses.

Dans un mode de réalisation, les formulations sont sous forme d'une solution injectable.

La préparation d'une composition selon l'invention présente l'avantage de pouvoir être réalisée par simple mélange d'une solution aqueuse d'insuline humaine A21G ou sous forme lyophilisée.

La composition du mélange est ajustée en excipients tels que glycérine, m-crésol et/ou phénol, chlorure de zinc, et polysorbate 20 (Tween^{®} 20). Cette addition peut être effectuée par ajout de solutions concentrées desdits excipients.

Dans un mode de réalisation, les compositions sont caractérisées en ce que lesdites compositions sont limpides à un pH de 7,4 et présentent une stabilité physique et chimique d'au moins un mois à 40°C.

Dans un mode de réalisation, les compositions sont caractérisées en ce que lesdites compositions présentent une stabilité physique et chimique supérieure à celle d'une insuline prandiale commerciale.

L'insuline et les insulines analogues peuvent être obtenues par des méthodes de technologies de l'ADN recombinante en bactéries telle que l'Escherichia Coli ou en levures telle que le Saccharomyces Cerevisiae (voir par exemple G. Walsh Appl. Microbiol. Biotechnol. 2005, 67, 151-159). Généralement, on produit une proinsuline qui est ensuite digérée par des enzymes telles que la trypsine et la carboxypetidase B pour obtenir la séquence souhaitée.

Pour la fabrication d'insuline humaine A21G, on code la proinsuline pour avoir la glycine en A21 et après digestion par la trypsine et la carboxypeptidase B, on obtient l'insuline souhaitée. Un mode opératoire est décrit par Kohn et al., dans Peptides 2007, 28, 935-948.

Dans un mode de réalisation, la composition selon l'invention est caractérisée en ce que l'insuline A21G est obtenue à partir de l'insuline humaine A21G, B31R, B32R (insuline glargine).

Dans un mode de réalisation, la composition selon l'invention est caractérisée en ce que l'insuline A21G est obtenue à partir de l'insuline humaine A21G, B31R, B32R (insuline glargine) mise en réaction avec la carboxypeptidase B de rat à un ratio insuline/carboxypeptidase compris entre 500 et 2000, à un pH compris de 7,5 à 8,5 et à une température comprise de 20 à 30 °C pendant 10 à 20 heures. Le produit peut ensuite être purifié. Cette purification peut être effectuée par chromatographie liquide.

Une voie de préparation de l'insuline humaine A21G peut donc être obtenue en enlevant les deux arginines de l'insuline glargine par digestion avec une carboxypeptidase B. Après digestion enzymatique, l'insuline humaine A21G peut être purifiée par chromatographie puis isolée par lyophilisation ou par cristallisation par des méthodes classiques.

### Description des figures

### Figure 1 :

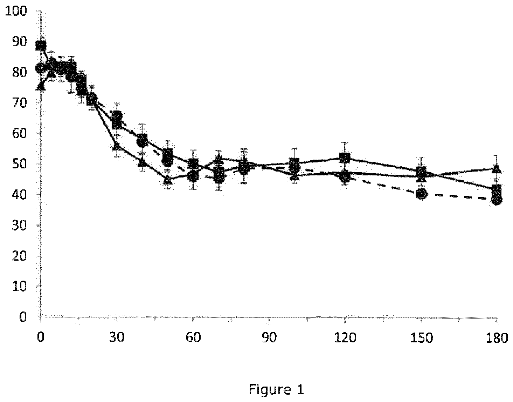

La figure 1 représente la pharmacodynamie de la composition B5-1 à t0 (c'est-à-dire conservée à 4°C) et à t12 semaines (composition B5-1 « vieillie » à 40°C sur 12 semaines) et de la composition B4, formulation commerciale d'insuline humaine Humulin^{®}.

Sur cette figure l'abscisse représente le temps après injection (en minutes) et l'ordonnée représente la glycémie sanguine (en mg/dL).

### Figure 1 :

Sont représentés à la Figure 1 les résultats de pharmacodynamie obtenus avec la composition B5-1 administrée à t0 (courbe tracée avec les carrés) et à t12 semaines à 40 °C (courbe tracée avec les triangles) en comparaison avec les résulats obtenus avec la composition B4 (insuline humaine de référence) (courbe tracée en pointillé avec les ronds).

Les exemples suivants illustrent, de manière non-limitative, l'invention.

### Exemples

### A Chimie

### Exemple A1: Préparation de l'insuline humaine A21G (insuline A21G)

De l'insuline glargine (5 g; Gan & Lee Pharmaceuticals) a été mélangée avec de l'enzyme carboxypeptidase B (Référence 08039852001 ; Sigma-Aldrich) à un ratio de 1/500 (w/w) à pH 8,0 (pH ajusté par ajout de tampon trishydroxyméthylaminométhane), la concentration en insuline glargine finale étant d'environ 4 mg/ml.

La solution a été laissée sous agitation douce à 25 °C pendant 17 h. Le mélange a ensuite été purifié par chromatographie liquide, dialysé contre de l'acide chlorhydrique 0,01 N puis lyophilisé.

L'insuline humaine A21G a été obtenue avec une pureté de 98 % et un rendement d'environ 90 %.

La masse molaire de l'insuline A21G mesurée par spectrométrie de masse (Maldi-Tof) est de 5752 Da.

### B Compositions

### Exemple B2 : Composition d'insuline analogue rapide (Humalog^{®}) à 100 U/mL

Cette composition est une solution commerciale d'insuline lispro commercialisée par la société ELI LILLY sous le nom de Humalog^{®}. Ce produit est une insuline analogue rapide. Les excipients dans Humalog^{®} sont le méta-crésol (3,15 mg/mL), le glycérol (16 mg/mL), le phosphate de disodium (1,88 mg/mL), l'oxyde de zinc (pour avoir 0,0197 mg d'ion zinc/mL), l'hydroxyde de sodium et l'acide chlorhydrique pour l'ajustement du pH (pH 7-7,8) et de l'eau.

### Exemple B3 : Composition d'insuline analogue lente (Lantus^{®}) à 100 U/mL

Cette composition est une solution commerciale d'insuline glargine commercialisée par la société SANOFI sous le nom de Lantus^{®}. Ce produit est une insuline analogue lente. Les excipients dans Lantus^{®} sont le chlorure de zinc (30 µg/mL), le m-crésol (2,7 mg/mL), le glycérol (20 mg/mL), le polysorbate 20 (16 µM), l'hydroxyde de sodium et l'acide chlorhydrique pour l'ajustement du pH (pH 4) et de l'eau.

### Exemple B4 : Composition d'insuline humaine (Humulin^{®} R) à 100 UI/mL

Cette composition est une solution commerciale d'insuline humaine de ELI LILLY vendue sous le nom d'Humulin^{®} R. Ce produit est une insuline humaine. Les excipients d'Humulin^{®} R sont le glycérol, le méta-crésol, l'hydroxyde de sodium et l'acide chlorhydrique pour l'ajustement du pH (pH 7,0-7,8) et de l'eau.

### Exemple B5: Préparation de compositions d'insuline A21G à des concentrations comprises entre 100 et 300 U/mL et d'excipients

Des compositions d'insuline humaine A21G ont été préparées selon le protocole suivant.

Une solution aqueuse d'insuline humaine A21G à pH 7,3 ± 0,2 est préparée à partir du lyophilisat obtenu à l'exemple A1 et d'une quantité en hydroxyde de sodium ou acide chlorhydrique nécessaire à l'ajustement du pH. Cette solution est ajoutée à un mélange de solutions concentrées des excipients (chlorure de zinc, conservateur, agent osmotique, trishydroxyméthylaminométhane, arginine). Une solution de tensioactif est ajoutée ensuite au mélange. Le pH est mesuré et ajusté si nécessaire entre 7,1 ± 0,1 et 8,0 ± 0,1 par ajout de solutions concentrées d'hydroxyde de sodium ou d'acide chlorhydrique. On obtient une solution limpide qui ne contient pas de particules visibles. Cette inspection est réalisée selon les recommandations des Pharmacopées Européenne (EP 2.9.20) et américaine (USP <790>). Le conservateur est soit le phénol (25 à 50 mM), soit le méta-crésol (25 mM). Les concentrations des solutions mères sont choisies de façon à obtenir une concentration en trishydroxyméthylaminométhane de 0 à 50 mM et/ou d'arginine de 0 à 50 mM dans la composition finale. La concentration de solution mère d'agent osmotique, ici de glycérol, est choisie de façon à obtenir une concentration de glycérol comprise entre 50 et 210 mM dans la composition finale. Le tensioactif utilisé peut être le Tween^{®} 20, à une concentration d'au moins 8 µM, ou le Poloxamer^{®} 188, à une concentration de 1,2 mg/mL, dans la composition finale. La concentration d'insuline humaine A21G, C_{IHA21G}, est comprise entre 100 et 300 U/mL. La concentration en chlorure de zinc (µM) correspond à 2 à 3 fois la concentration d'insuline humaine A21G (U/ml), soit C_{ZnCl2} (µM) = 2-3 C_{IHA21G} (U/ml).

Ces compositions sont présentées dans le tableau 1 ci-après.

### Exemple B6: Préparation de compositions d'insuline humaine A21G à des concentrations comprises entre 100 et 300 U/mL (correspondant à 3,5 et 10,5 mg/ml) et d'excipients à pH 4

Ces compositions ont été réalisée selon le protocole décrit à l'exemple B5 à partir d'une solution aqueuse d'insuline humaine A21G à pH 4,0 ± 0,2, préparée à partir du lyophilisat obtenu à l'exemple A1 et d'une quantité en hydroxyde de sodium ou en acide chlorhydrique permettant l'ajustement de pH. La concentration en trishydroxyméthylaminométhane est comprise entre 0 et 50 mM dans la composition finale. Les concentrations des autres excipients sont similaires à celles mentionnées à l'exemple B5.

Ces compositions sont présentées dans le tableau 1 ci-après.

**Tableau 1 : Compositions d'insuline A21G préparées selon les protocoles B5 (B5-1 à B5-13) et B6 (B6-1 à B6-3)**

| **Compo sition** | **Insuline (mq/mL)** | **Tris (mM)** | **Additif** | **Tensioactif** | **[Zn] (µM)** | **Glycérol (mM)** | **pH (±0,1)** |
|---|---|---|---|---|---|---|---|
| B5-1 | 3,5 | 10 | Phénol (50 mM) | Tween^{®} 20 (8 µM) | 230 | 200 | 7,4 |
| B5-2 | 3,5 | 10 | Phénol (50 mM) | Poloxamer^{®} 188 (1,2 mg/mL) | 230 | 184 | 7,4 |
| B5-3 | 3,5 | - | m-crésol (25 mM) | Poloxamer^{®} 188 (1,2 mg/mL) | 300 | 184 | 7,4 |
| B5-4 | 3,5 | 10 | Phénol (50 mM) | Tween^{®} 20 (8 µM) | 230 | 200 | 7,0 |
| B5-5 | 3,5 | 10 | Phénol (50 mM) | Tween^{®} 20 (8 µM) | 230 | 200 | 7,2 |
| B5-6 | 3,5 | 10 | Phénol (50 mM) | Tween^{®} 20 (8 µM) | 230 | 200 | 7,5 |
| B5-7 | 3,5 | 10 | Phénol (50 mM) | Tween^{®} 20 (8 µM) | 230 | 200 | 8,0 |
| B5-8 | 10,5 | - | m-cresol (25 mM) | Poloxamer^{®} 188 (1,2 mg/mL) | 900 | 184 | 7,4 |
| B5-9 | 3,5 | - | Phénol (50 mM) Arginine (50 mM) | Tween^{®} 20 (8 µM) | 230 | 184 | 7,4 |
| B5-10 | 7 | 10 | Phénol (50 mM) | Tween^{®} 20 (8 µM) | 460 | 184 | 7,4 |
| B5-11 | 10,5 | 10 | Phénol (50 mM) | Tween^{®} 20 (8 µM) | 690 | 184 | 7,4 |
| B5-12 | 3,5 | 50 | Phénol (50 mM) Arginine (40 mM) | Tween^{®} 20 (8 µM) | 230 | 60 | 7,4 |
| B6-1 | 3,5 | - | m-cresol (25 mM) | Poloxamer^{®} 188 (1,2 mq/mL) | 300 | 184 | 4,0 |
| B6-2 | 3,5 | - | m-cresol (25 mM) | Poloxamer^{®} 188 (1,2 mg/mL) | - | 184 | 4,0 |
| B6-3 | 10,5 | - | m-cresol (25 mM) | Tween^{®} 20 (8 µM) | - | 184 | 4,0 |
| B5-13 | 10,5 | 10 | Phénol (50 mM) | Tween^{®} 20 (8 µM) | 690 | 200 | 7,4 |

### C Stabilité

### Exemple C1 : Etude de stabilité physique à 50 °C des compositions préparées en comparaison avec des insulines commerciales à une concentration de 100 U/mL en vials (flacons).

Au moins 5 vials de 3 mL remplis avec 1 mL de composition sont placés verticalement dans une étuve maintenue à 50 °C. Les vials sont inspectés visuellement avec une fréquence au moins hebdomadaire afin de détecter l'apparition de particules visibles ou d'une turbidité. Cette inspection est réalisée selon les recommandations des Pharmacopées Européenne (EP 2.9.20) et américaine (USP <790>): les vials sont soumis à un éclairage d'au moins 2000 Lux et sont observés face à un fond blanc et un fond noir. Le nombre de semaines de stabilité correspond à la durée pendant laquelle au moins la moitié des vials reste limpide et ne contient pas de particules visibles à l'inspection.

Les résultats de stabilité physique obtenus avec différentes compositions sont présentés dans le tableau ci-après.

**Tableau 2 : Résultats d'études de stabilité physique à 50 °C des compositions B5-1 à B5-3, B5-9, B5-12, B6-1 à B6-3 et B2 à B4.**

| **Composition** | **Insuline** | **Concentration en insuline (mg/mL)** | **pH** | **50 °C** |
|---|---|---|---|---|
| B5-1 | A21G | 3,5 | 7,4 | Pas de particules à 15 semaines |
| B5-2 | A21G | 3,5 | 7,4 | Pas de particules à 15 semaines |
| B5-3 | A21G | 3,5 | 7,4 | Pas de particules à 8 semaines |
| B5-9 | A21G | 3,5 | 7,4 | Pas de particules à 9 semaines |
| B5-12 | A21G | 3,5 | 7,4 | Pas de particules à 6 semaines |
| B6-1 | A21G | 3,5 | 4,0 | Particules à 1 semaine |
| B6-2 | A21G | 3,5 | 4,0 | Particules à 1 semaine |
| B6-3 | A21G | 3,5 | 4,0 | Particules à 4 semaines |
| B2 (Humalog^{®}) | Insuline lispro | 3,5 | 7,4 | Particules à 1 semaine |
| B3 (Lantus^{®}) | Insuline glargine | 3,6 | 4,0 | Particules à 1 semaine |
| B4 (Humulin^{®} R) | Insuline humaine | 3,5 | 7,4 | Particules à 2 semaines |

Les compositions d'insuline humaine A21G à pH 4,0 (B6-1 à B6-3) et les formulations commerciales (B2 à B4) sont moins stables à 50 °C que les compositions contenant de l'insuline humaine A21G à pH 7,4 (B5-1 à B5-3, B5-9, et B5-12). Ces compositions (B5-1 à B5-3, B5-9, et B5-12) permettent d'obtenir une stabilité physique d'au moins 6 semaines à 50 °C.

### Exemple C2 : Etude de stabilité chimique à 40 °C et 50 °C.

Les compositions, présentées dans le tableau suivant, sont placées dans une étuve maintenue à 40 °C ou 50 °C.

Les compositions sont analysées à 4, 8, 12 semaines à 40 °C et à 2 semaines à 50 °C. À chaque échéance, un prélèvement est analysé par RP-HLPC-UV (214 nm) avec une colonne C₁₈ et un gradient d'élution tampon phosphate/acétonitrile en présence d'heptanesulfonate de sodium pour déterminer par pourcentage surfacique la proportion des produits déamidés dB3/disoB3 ainsi que des autres impuretés (notées impuretés totales). À chaque échéance, un prélèvement est aussi analysé par SE-HPLC-UV (276nm) avec une colonne en silice enrobée avec des pores de 80 Å et une phase mobile eau/acétonitrile/acide trifluoroacétique pour déterminer par pourcentage surfacique la proportion des produits de haut poids moléculaire (HMWP).

Les résultats sont donnés en pourcentage dans le tableau ci-dessous.

**Tableau 3 : Résultats des stabilités chimiques des solutions B5-1 à B5-3, B5-9, et B5-12 et de compositions commerciales d'insuline humaine Humulin^{®} R**

| **Composition** | **Insuline (mg/mL)** | **Produits de déqradation** | **TO** | **40 °C 4S** | **40 °C 8S** | **40 °C 12S** | **50 °C 2S** |
|---|---|---|---|---|---|---|---|
| B5-1 | A21G | dB3/disoB3 | 0,4 | 1,4 | 2,8 | 4,2 | 1,8 |
| | (3,5) | HMWP | 0,1 | 0,3 | 0,7 | 0,9 | 0,3 |
| | | Imp. Totales^{∗} | 0,3 | 1,9 | 3,7 | 5,0 | 2,2 |
| B5-2 | A21G | dB3/disoB3 | 0,1 | - | - | - | 2,0 |
| | (3,5) | HMWP | 0,2 | | | | 0,5 |
| | | Imp. Totales^{∗} | 0,3 | | | | 2,5 |
| B5-3 | A21G | dB3/disoB3 | 0,2 | 3,0 | 5,7 | 8,7 | 4,2 |
| | (3,5) | HMWP | 0,1 | 0,4 | 1,0 | 1,9 | 0,5 |
| | | Imp. Totales^{∗} | 0,6 | 1,4 | 4,1 | 5,2 | 2,3 |
| B5-9 | A21G | dB3/disoB3 | 0,1 | - | - | - | 1,8 |
| | (3,5) | HMWP | 0,1 | | | | 0,1 |
| | | Imp. Totales^{∗} | 0,3 | | | | 2,5 |
| B5-12 | A21G | dB3/disoB3 | 0,1 | 1,4 | 2,8 | 4,1 | 1,6 |
| | (3,5) | HMWP | 0,1 | 0,2 | 0,2 | 0,3 | 0,1 |
| | | Imp. Totales^{∗} | 0,4 | 2,3 | 4,3 | 5,6 | 2,5 |
| B4 | Insuline humaine (3,5) | dB3/disoB3 | 2,5 | 11,7 | - | - | 6,4 |
| | | HMWP | 0,3 | 2,1 | - | - | 3,4 |
| | | Imp. Totales^{∗} | 1,4 | 12,8 | - | - | 6,5 |

| | | | | | | | |
|---|---|---|---|---|---|---|---|
| « ^{∗} » par « imp. totales » on entend toutes les impuretés hors dB3/disoB3 et HMWP « - » signifie non mesuré | | | | | | | |

La composition commerciale Humulin^{®} R (B4) est analysée après 4, 8 et 12 semaines à 40 °C et après 2 semaines à 50 °C. A ces échéances, cette composition ne satisfait plus les spécifications de la Pharmacopée Européenne en termes de stabilité physique (voir ci-dessus), ni en termes de pourcentage d'impuretés. Les formulations B5-1 à B5-3, B5-9 et B5-12 sont bien plus stables.

### Exemple C3 : Effet du pH sur la stabilité physique et chimique de compositions comprenant de l'insuline humaine A21G

Les formulations B5-4 à B5-7 ont une composition identique à celle de la formulation B5-1, sauf pour le pH.

**Tableau 4 : Résultats de l'effet du pH sur la stabilité physique et chimique des solutions B5-4 à B5-7**

| **Composition** | **pH** | **Vial 40 °C** | **Produits de dégradation après 4 semaines à 40 °C (par rapport à t0)** | | | |
|---|---|---|---|---|---|---|
| B5-4 | 7,0 ± **0,1** | Particules observées tout de suite à t0 | - | | | |
| B5-5 | 7,2 ± 0,1 | Pas de particules à 11 semaines | | dB3/disoB3 | | 1,3 % |
| | | | | | HMWP | 0,1 % |
| | | | | | Imp. totales^{∗} | 1,8 % |
| B5-6 | 7,5 ± 0,1 | Pas de particules à 11 semaines | | dB3/disoB3 | | 1,4 % |
| | | | | | HMWP | 0,2 % |
| | | | | | Imp. totales^{∗} | 1,9 % |
| B5-7 | 8,0 ± 0,1 | Pas de particules à 11 semaines | | dB3/disoB3 | | 1,6 % |
| | | | | | HMWP | 0,3 % |
| | | | | | Imp. totales^{∗} | 2,4 % |

| | | | | | | |
|---|---|---|---|---|---|---|
| ^{∗} par « impuretés totales » on entend toutes les impuretés hors dB3/disoB3 et HMWP « - » signifie non mesuré | | | | | | |

Les compositions présentent une bonne stabilité entre 7,2 et 8,0. Il est à noter qu'une stabilité encore meilleure à 40 °C est obtenue dans une gamme de pH compris entre 7,2 et 7,5.

### Exemple C4 : Stabilité physique et chimique de l'insuline humaine A21G à 100 et 300 U/mL (soit 3,5 et 10,5 mg/ml), à pH 7,4.

**Tableau 5 : Résultats des stabilité physiques à 50 °C des compositions à 100 et 300 U/mL d'insuline A21G (B5-3 et B5-8).**

| **Formulation** | **Insuline A21G (mg/mL)** | **50 °C** |
|---|---|---|
| B5-3 | 3,5 | Pas de particules à 8 semaines |
| B5-8 | 10,5 | Pas de particules à 4 semaines Particules à 5 semaines |

**Tableau 6 : Résultats des stabilités chimiques à 40 et 50 °C des compositions à 100 et 300 U/mL d'insuline A21G (B5-3 et B5-8).**

| **Compo sition** | **Insuline A21G (mg/mL)** | **Produits de dégradation** | **TO** | **40°C 4S** | **40°C 8S** | **40°C 12S** | **50°C 2S** |
|---|---|---|---|---|---|---|---|
| B5-3 | 3,5 | dB3/disoB3 | 0,2 | 3,0 | 5,7 | 8,9 | 4,2 |
| | | HMWP | 0,1 | 0,4 | 1,0 | 1,9 | 2,3 |
| | | Imp. Totales | 0,6 | 1,4 | 4,1 | 5,2 | 0,5 |
| B5-8 | 10,5 | dB3/disoB3 | 0,4 | 1,9 | 3,8 | 5,9 | 2,7 |
| | | HMWP | 0,1 | 0,2 | 0,5 | 0,9 | 2,0 |
| | | Imp. Totales | 1,2 | 1,3 | 3,1 | 3,9 | 0,4 |

| | | | | | | | |
|---|---|---|---|---|---|---|---|
| ^{∗} par « imp. totales » on entend toutes les impuretés hors dB3/disoB3 et HMWP | | | | | | | |

Les résultats sont exprimés en pourcentage dans le tableau ci-dessous.

Les résultats présentés ci-dessus montrent que la composition B5-8 (U300) présente une bonne stabilité physique et une excellente stabilité chimique.

### Exemple C5 : Stabilité physique et chimique de l'insuline humaine A21G à 100 et 300 U/mL (soit 3,5 et 10,5 mg/ml) en cartouches (3 mL), à 40°C.

Les compositions B5-1 et B5-13 de l'insuline humaine A21G sont insérées dans des cartouches de 3 mL et placées dans une étuve à 40 °C en conditions statiques. Les cartouches sont inspectées visuellement toutes les deux semaines afin de détecter l'apparition de particules visibles ou d'une turbidité. Cette inspection est réalisée selon les recommandations des pharmacopées Européenne (EP 2.9.20) et américaine (USP <790>) décrites pour des solutions en vial. Ainsi, les cartouches sont soumises à un éclairage d'au moins 2000 Lux et sont observées face à un fond blanc et un fond noir. Le nombre de semaines de stabilité correspond à la durée pendant laquelle au moins la moitié des cartouches reste limpide et ne contient pas de particules visibles à l'inspection.

Les cartouches sont analysées à 4, 8 et 12 semaines à 40 °C. À chaque échéance, une cartouche est prélevée et un aliquot est analysé par RP-HLPC-UV (214 nm) avec une colonne C₁₈ et un gradient d'élution tampon phosphate/acétonitrile en présence d'heptanesulfonate de sodium pour déterminer par pourcentage surfacique la proportion des produits déamidés dB3/disoB3 ainsi que des autres impuretés (notées impuretés totales). À chaque échéance, un aliquot est aussi analysé par SE-HPLC-UV (276nm) avec une colonne en silice enrobée avec des pores de 80 Å et une phase mobile eau/acétonitrile/acide trifluoroacétique pour déterminer par pourcentage surfacique la proportion des produits de haut poids moléculaire (HMWP).

Les résultats sont donnés en pourcentage dans le tableau ci-dessous.

**Tableau 6a : Résultats des stabilité physiques à 40 °C des compositions à 100 et 300 U/mL d'insuline A21G (B5-1 et B5-13).**

| **Composition** | **Insuline A21G (mg/mL)** | **40 °C** |
|---|---|---|
| B5-1 | 3,5 | Pas de particules à 12 semaines |
| B5-13 | 10,5 | Pas de particules à 10 semaines |

**Tableau 6b : Résultats des stabilités chimiques des solutions B5-1 et B5-13.**

| **Composition** | **Insuline (mg/mL)** | **Produits de dégradation** | **T0** | **40 °C 4S** | **40 °C 8S** | **40 °C 11S** | **40 °C 12S** |
|---|---|---|---|---|---|---|---|
| B5-1 | A21G | dB3/disoB3 | 0,1 | 1,4 | 2,6 | - | 3,8 |
| | (3,5) | HMWP | 0,2 | 0,3 | 0,4 | - | 0,7 |
| | | Imp. Totales^{∗} | 0,6 | 1,9 | 3,1 | - | 4,5 |
| B5-13 | A21G 10,5) | dB3/disoB3 | 0,3 | - | - | 3,2 | - |
| | | HMWP | 0,2 | - | - | 0,5 | - |
| | | Imp. Totales^{∗} | 0,4 | - | - | 4,0 | - |

| | | | | | | | |
|---|---|---|---|---|---|---|---|
| « ^{∗} » par « imp. totales » on entend toutes les impuretés hors dB3/disoB3 et HMWP | | | | | | | |

Les résultats sont exprimés en pourcentage dans le tableau ci-dessous.

Les résultats présentés ci-dessus montrent que les compositions B5-1 (U100) et B5-13 (U300) présentent une excellente stabilité physique et chimique.

### D Pharmacodynamie

### Exemple D1 : Protocole de mesure de la pharmacodynamie des solutions d'insuline

Des porcs domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose.

L'injection des formulations d'insuline M1 ou d'insuline humaine à la dose de 0,2 UI/kg est réalisée en sous-cutané au niveau du flanc de l'animal à l'aide d'un stylo à insuline (Novo, Sanofi ou Lilly) équipé d'une aiguille 31 G.

Des prélèvements sanguins sont ensuite réalisés aux temps suivants : 4, 8, 12, 16, 20, 30, 40, 50, 60, 70, 80, 100, 120, 150 et 180 minutes. Après chaque prélèvement, le cathéter est rincé avec une solution diluée d'héparine. Une goutte de sang est prélevée pour déterminer la glycémie au moyen d'un glucomètre. Les courbes moyennes de glycémie, exprimée mg/dL, sont ensuite tracées.

**Exemple D2** : Résultats de pharmacodynamie de la solution d'insuline A21G B5-1 au temps zéro (t0, c'est-à-dire conservée à 4°C) et après 12 semaines à 40 °C et d'une solution d'insuline humaine.

**Tableau 7 : Compositions utilisées pour la pharmacodynamie**

| **Composition** | **Insuline humaine Insuline A21G (U/mL) humaine (U/mL)** | | **Nombre de porcs** |
|---|---|---|---|
| B5-1 | 100 | - | 12 |
| B4 | - | 100 | 11 |

Les résultats de pharmacodynamie obtenus avec la composition B5-1 sont illustrés dans la FIG. 1. L'analyse de ces profils indique que des actions hypoglycémiantes similaires sont obtenues avec la composition B5-1 administrée à t0 (courbe tracée avec les carrés) et à t12 semaines à 40 °C (courbe tracée avec les triangles). D'autre part, la composition B5-1 administrée à t0 ou à t12 semaines à 40 °C présente une action hypoglycémiante similaire à celle obtenue avec la composition B4 (insuline humaine de référence) (courbe tracée en pointillé avec les ronds).

## Revendications

1. Composition sous forme d'une solution aqueuse injectable, dont le pH est compris entre 7,2 et 8,0 (7,2 ≤ pH ≤ 8,0) comprenant au moins de l'insuline humaine A21G.

2. Composition selon la revendication 1, **caractérisée en ce que** la concentration en insuline humaine A21G est comprise entre 40 et 1000 U/mL (40 U/mL ≤ concentration en insuline humaine A21G ≤ 1000 U/mL).

3. Composition selon la revendication 1 ou 2, **caractérisée en ce que** la concentration en insuline humaine A21G est de 100 U/mL.

4. Composition selon l'une quelconque des revendications 1 à 3, **caractérisée en ce que** la concentration en insuline humaine A21G est de 300 U/mL.

5. Composition selon l'une quelconque des revendications 1 à 4, **caractérisée en ce que** la concentration en sel de zinc est comprise entre 50 et 600 µM pour 100 U/mL d'insuline A21G.

6. Composition selon l'une quelconque des revendications précédentes, **caractérisée en ce que** la composition comprend en outre un conservateur phénolique, notamment choisi parmi le phénol et le m-crésol.

7. Composition selon la revendication 6, **caractérisée en ce que** la concentration en conservateur phénolique est comprise entre 15 et 100 mM.

8. Composition selon l'une quelconque des revendications précédentes, **caractérisée en ce qu'**elle comprend en outre un tensioactif.

9. Composition selon la revendication 8, **caractérisée en ce que** le tensioactif est choisi parmi les polysorbates.

10. Composition selon la revendication 9, **caractérisée en ce que** le tensioactif est choisi parmi le polysorbate 20 ou « Tween^{®} 20 ».

11. Composition selon l'une quelconque des revendications précédentes, **caractérisée en ce qu'**elle comprend en outre de l'arginine.

12. Composition selon l'une quelconque des revendications précédentes, **caractérisée en ce qu'**elle comprend du trishydroxyméthylaminométhane.

13. Composition selon la revendication 12, **caractérisée en ce qu'**elle comprend du trishydroxyméthylaminométhane à une concentration comprise de 2 à 100 mM (2 mM ≤ concentration en trishydroxyméthylaminométhane ≤ 100 mM).

14. Composition selon l'une quelconque des revendications précédentes destinée à être utilisée dans une méthode de traitement du diabète **caractérisée en ce qu'**elle est administrée en bolus avant les repas.

15. Composition selon l'une quelconque des revendications précédentes destinée à être utilisée dans une méthode de traitement du diabète **caractérisée en ce qu'**elle est administrée en tant qu'insuline prandiale.

## Patentansprüche

1. Zusammensetzung in der Form einer wässrigen injizierbaren Lösung, deren pH-Wert zwischen 7,2 und 8,0 liegt (7,2 ≤ pH-Wert ≤ 8,0), umfassend mindestens ein Humaninsulin A21G.

2. Zusammensetzung nach Anspruch 1, **dadurch gekennzeichnet, dass** die Konzentration von Humaninsulin A21G zwischen 40 und 1000 U/mL liegt (40 U/mL ≤ Konzentration von Humaninsulin A21G ≤ 1000 U/mL).

3. Zusammensetzung nach Anspruch 1 oder 2, **dadurch gekennzeichnet, dass** die Konzentration von Humaninsulin A21G 100 U/mL beträgt.

4. Zusammensetzung nach einem der Ansprüche 1 bis 3, **dadurch gekennzeichnet, dass** die Konzentration von Humaninsulin A21G 300 U/mL beträgt.

5. Zusammensetzung nach einem der Ansprüche 1 bis 4, **dadurch gekennzeichnet, dass** die Konzentration von Zinksalz zwischen 50 und 600 µM für 100 U/mL Insulin A21G liegt.

6. Zusammensetzung nach einem der vorhergehenden Ansprüche, **dadurch gekennzeichnet, dass** die Zusammensetzung weiterhin ein phenolisches Konservierungsmittel umfasst, das insbesondere aus Phenol und m-Kresol ausgewählt ist.

7. Zusammensetzung nach Anspruch 6, **dadurch gekennzeichnet, dass** die Konzentration von phenolischem Konservierungsmittel zwischen 15 und 100 mM liegt.

8. Zusammensetzung nach einem der vorhergehenden Ansprüche, **dadurch gekennzeichnet, dass** sie weiterhin ein Tensid umfasst.

9. Zusammensetzung nach Anspruch 8, **dadurch gekennzeichnet, dass** das Tensid aus Polysorbaten ausgewählt ist.

10. Zusammensetzung nach Anspruch 9, **dadurch gekennzeichnet, dass** das Tensid aus Polysorbat 20 oder "Tween" 20 ausgewählt ist.

11. Zusammensetzung nach einem der vorhergehenden Ansprüche, **dadurch gekennzeichnet, dass** sie weiterhin Arginin umfasst.

12. Zusammensetzung nach einem der vorhergehenden Ansprüche, **dadurch gekennzeichnet, dass** sie Trishydroxymethylaminomethan umfasst.

13. Zusammensetzung nach Anspruch 12, **dadurch gekennzeichnet, dass** sie Trishydroxymethylaminomethan in einer Konzentration umfasst, die 2 bis 100 mM umfasst (2 mM ≤ Konzentration von Trishydroxymethylaminomethan ≤ 100 mM).

14. Zusammensetzung nach einem der vorhergehenden Ansprüche, die dazu vorgesehen ist, in einem Verfahren zur Behandlung von Diabetes verwendet zu werden, **dadurch gekennzeichnet, dass** sie in einem Bolus vor einer Mahlzeit verabreicht wird.

15. Zusammensetzung nach einem der vorhergehenden Ansprüche, die dazu vorgesehen ist, in einem Verfahren zur Behandlung von Diabetes verwendet zu werden, **dadurch gekennzeichnet, dass** sie als prandiales Insulin verabreicht wird.

## Claims

1. Composition in the form of an injectable aqueous solution, the pH of which is between 7.2 and 8.0 (7.2 ≤ pH ≤ 8.0) comprising at least A21G human insulin.

2. Composition according to claim 1, **characterized in that** the concentration of A21G human insulin is between 40 and 1000 U/mL (40 U/mL ≤ concentration of A21G human insulin ≤ 1000 U/mL).

3. Composition according to claim 1 or 2, **characterized in that** the concentration of A21G human insulin is 100 U/mL.

4. Composition according to any one of claims 1 to 3, **characterized in that** the concentration of A21G human insulin is 300 U/mL.

5. Composition according to any one of claims 1 to 4, **characterized in that** the zinc salt concentration is between 50 and 600 µM per 100 U/mL of A21G insulin.

6. Composition according to any one of the preceding claims, **characterized in that** the composition further comprises a phenolic preservative, in particular chosen from phenol and m-cresol.

7. Composition according to claim 6, **characterized in that** the concentration of phenolic preservative is between 15 and 100 mM.

8. Composition according to any one of the preceding claims, **characterized in that** it further comprises a surfactant.

9. Composition according to claim 8, **characterized in that** the surfactant is chosen from polysorbates.

10. Composition according to claim 9, **characterized in that** the surfactant is chosen from polysorbate 20 or "Tween^{®} 20".

11. Composition according to any one of the preceding claims, **characterized in that** it further comprises arginine.

12. Composition according to any one of the preceding claims, **characterized in that** it comprises trishydroxymethylaminomethane.

13. Composition according to the preceding claim 12, **characterized in that** it comprises trishydroxymethylaminomethane at a concentration between 2 and 100 mM (2 mM ≤ trishydroxymethylaminomethane concentration ≤ 100 mM).

14. Composition according to any one of the preceding claims, intended for use in a method for treating diabetes, **characterized in that** it is administered as a bolus before meals.

15. Composition according to any one of the preceding claims, intended for use in a method for treating diabetes, **characterized in that** it is administered as prandial insulin.
